# EUROPEAN PATENT APPLICATION

(11) **EP 4 008 791 A1**
(43) Date of publication of application: **08.06.2022**
(21) Application number: 20843787.1
(22) Date of filing: 22.07.2020
(51) Int. Cl.: C12Q 1/6844, C12Q 1/6888

(54) **MOLECULAR DIAGNOSTIC KIT FOR DETECTING NUCLEOTIDE SEQUENCES AND METHODS FOR DETECTING INFECTIOUS AGENTS USING SAID KIT**

(30) Priority: 23.07.2019 AR P190102073
(71) Applicant: Melossi Jiménez, Andrés, Santiago, 7500000 (CL); Laboratorio Pablo Cassara S.r.L., Buenos Aires, C1408GBV (AR); Unifarma S.A., Buenos Aires, 1427 (AR); Consejo Nacional De Investigaciones Cientificas Y Tecnicas (Conicet), Buenos Aires, 1425 (AR)
(72) Inventor: CARRILLO, Carolina, 1406 Caba (AR); VOJNOV, Adrian Alberto, Depto B Caba (AR); LAROCCA, Luciana, Buenos Aires (AR); STOLOWICZ, Fabiana, 1431 Caba (AR); WERBAJH, Santiago Enrique, 1101 Caba (AR)
(74) Representative: Durán-Corretjer, S.L.P.
(86) International application number: PCT/CL2020/050079
(87) International publication number: WO 2021/012063

(57) **Abstract**

Molecular-based diagnostic kits and methods for detecting nucleotide sequences of DNA, DNA copy and RNA of infectious agents, transgenes, alleles or non-encoding sequences, with no initial isolation are provided. The kit comprises at least a first calibrated dropper bottle with an anti-contamination system containing a solution of polymerase enzyme, chaotropic agents, salts and deoxyribonucleotides; a second calibrated dropper bottle with an anti-contamination system containing a set of at least 4 primers, part of the sequences being preserved in the nucleic acid fragment to be detected; a dropper bottle containing a mixture of developing reagents; a carrier to hold the sample; at least one LFD developing system, colorimetric or fluorogenic reaction; positive and negative controls. The polymerase enzyme is selected from Bst enzymes formed by DNA polymerase/helicase, with no exonuclease activity. The set of primers is designed from gene sequences to be detected, whether of deoxyribonucleic or ribonucleic nature.

## Description

### FIELD OF THE INVENTION

The invention relates to an easy-to-use molecular-based diagnostic kit for detection of DNA, DNA copy and/or RNA nucleotide sequences from pathogenic agents whether they are of protozoan, viral or bacteriologic origin, or else for detection of either transgenic sequences or genome itself that does not require prior isolation of the DNA or RNA to be detected. In addition, the kit is capable of detecting the sequence of interest in a great variety of matrices, such as animal or vegetal tissues and/or samples, such as insects, urine, blood, serum, tears, mucus, leaves, stems, etc.

### BACKGROUND

At present, most methods for detection of transgenic sequences or genome itself are either molecular-based, or else indirect by evaluation of a phenotypic function or trait acquired, lost or modified. On the other hand, methods for diagnosis of infections commercially available are of serologic basis; that is, they are based on detection of antibodies produced by the infected body in response to the presence of the infectious agent. Said methods have 84-98% sensitivity and they exhibit great variation also in specificity, costs and/or operative complexity. The present serologic methods are not effective in the case of human congenital Infections until about 9 months of age have elapsed, since they detect antibodies that are present in the blood stream, and these may be maternal antibodies that have passed through the placenta no matter whether the infectious agent has been transmitted or not to the neonate. Several studies reveal that 9 months is sufficient time to ensure clearance of maternal antibodies transferred during pregnancy.

Serologic methods can also exhibit anomalous behavior in immunocompromised subjects, whose immune response is altered.

Another condition in which serologic methods do not prove effective is in the follow-up of an infection during and after treatment, for the infectious agent may have been cleared but the reaction will continue to be positive as long as the antibodies against said agent are preserved in any sample in which antibodies may be present.

This is the inconvenience to test infections through the antibodies causing them: the delay between infectious focus elimination and antibody elimination.

Due to these limitations, in cases of congenital infections or in immunocompromised subjects, where high probabilities of obtaining false results prevail, other techniques are employed, such as the following:
i) Selective culture, which is an expensive, time-consuming technique involving biological risks such as handling of pathogen samples, preservation and growing thereof, thereby extending the pathogen population and therefore the amount of infectious agents, while also exhibiting very variable sensitivity.
ii) For certain types of infectious agents, microscope observation is employed, either directly or by concentration enrichment, these techniques having very variable sensitivity depending on the agent, infectious load, agent location, sample type, operator's skills, etc.
iii) Molecular methods, such as those of the present invention, which are based on pathogen genetic matter detection. Amplification techniques by means of Polymerase Chain Reaction (PCR) and variants such as "Nested PCR" and "Real Time - PCR" are considered more sensitive and specific than those previously mentioned. However, due to its intrinsic characteristics, this technique requires complex infrastructure and equipment, as well as qualified human resources.

The type of equipment required for molecular cycling methods have become more sophisticated, for which reason they improve their sensitivity at the cost of turning more expensive, either in the acquisition of equipment or in its maintenance and, in many cases, evidencing a shorter useful half-life. On the other hand, many methods for PCR detection and related techniques are 'in house' and require the handling of various reagents or else the preparation, also 'in house', of mixtures which stability is variable and/or indefinite and, therefore, cannot be stored, for which reason once they are prepared, they must be used at short term. All these preparations must be carried out under controlled environments to avoid contamination with amplicons from previous reactions and require the skilled use of calibrated micropipettes, tips with filters, etc., by staff specially qualified to such end. Even under controlled conditions, there exists contamination risk, which requires stopping the reactions, disposing contaminated reagents, decontaminating the area and reinitiating preparation and amplification actions.

In view of this, given the costs and general complexity and the sensitivity loss risks resulting from decalibration of equipment or pipettes or the appearance of false positives due to contamination, its applicability is restricted, in general, to a reduced number of diagnostic and/or investigation laboratories. Diagnostic kits are available in the prior art that detect DNA or DNA copy or of RNA fragments of certain length by means of a technology similar to that of this invention: the use of a polymerase enzyme that does not require successive changes in temperature to be able to advance in the reaction.

Patent US 5830714 A by Molecular Biology Resources, Inc. discloses a "Biologically active fragment of Bacillus stearothermophilus DNA polymerase", and it is directed to an isolated and purified DNA that encoding a biologically active fragment of a thermostable full length DNA polymerase I enzyme of Bacillus stearothermophilus. More particularly, it relates to a DNA encoding an approximately 66,000 Dalton DNA polymerase that lacks 273 amino acids from the N-terminus of the approximately 96,000 Dalton B. stearothermophilus DNA polymerase I, and to the protein encoded thereby which has been designated the B. stearothermophilus DNA polymerase I fragment. Said enzyme fragment is useful in DNA sequencing, cDNA preparations, thermophilic Strand Displacement Amplification and other molecular biology applications.

Patent US 8993298 B1 by New England Biolabs, Inc. et al discloses "DNA polymerases." Novel proteins having DNA polymerase are described which have improved synthesis properties over Bst polymerase such as for example enhanced reverse transcriptase activity.

Patent CN 102925548 B by the Sichuan Agricultural University discloses "Actinobacillus pleuropneumoniae LAMP kit and application method thereof". It relates to a kit for detecting A. faecalis, a porcine infectious agent and a method of use thereof. The kit comprises 50mul of 8 U/µl BstDNA polymerase, 125 µl of a BstDNA polymerase buffer solution, 100 µl of 0.1 mol/I MgSO4, 100 µl of 12.5 mmol/l betaine, 100 µl of 17.5 mmol/l dNTP. Said kit can detect trace amount of various serotypes of A. pneumoniae in a 63-64 ºC constant-temperature container. Though the method does not require cycling equipment, it does need the provision of various reagents, which involves handling of pipettes and their tips. In addition, the use of the kit implies a step - which has the advantage of being simple - of purifying the DNA that shall be used as template. Even with these requirements, it is proposed as a kit than can meet the need of carrying out "in situ" tests of porcine ampullary infectious bacillus. It can be used for import and export quarantines, food hygiene departments, and animal breeding farms.

It can further be used in "in situ" tests, especially suitable for field and real time detection of Actinobacillus pleuropneumoniae in pig-based veterinary workers.

### SUMMARY OF THE PRESENT INVENTION

The present invention relates to an extremely easy-to-use molecular-based diagnostic kit for detection of DNA and/or RNA fragments from pathogenic agents whether they are of protozoan, viral or bacteriologic origin, or else for detection of nucleotide sequences, for example, transgenes, alleles or non-encoding sequences. Functional templates for molecular amplification are DNA, DNA copy and RNA nucleotide sequences of certain length, regardless of their purity degree, derived from different biological matrices of urine, blood, serum, tears, mucus, leaves, etc.

Contrary to the prior art, with the present invention, high sensitivity detection of DNA, DNA copy and RNA nucleotide sequences has unexpectedly been achieved, with no need of prior preparation or isolation of genetic material, or the use of a thermal cycler to obtain multiplication reaction of selected amplicons, or the use of pipettes and their tips while employing the kit.

This kit substantially simplifies detection of pathogenic agents or the presence of transgenic, allelic or non-encoding nucleotide sequences, maintaining high sensitivity appropriate to that end. The provision of the kit allows for its direct use, because it does not require prior preparation of ingredients since the sample is placed directly on a filter paper or reaction tube. It does not require any laboratory equipment such as a thermal cycler, developing equipment o any equipment for reading results, nor of laboratory instruments, for example pipettes, or the addition of multiple ingredients because reagent mixtures have been developed to directly detect DNA or DNA copy fragments on the sample not yet purified.

Either the formulation of mixtures as well as their presentation allows for keeping them stable under cooled conditions at 2 - 8 degrees (common refrigerator) for periods of time of at least 18 months. The present kit meets the particular need of sensitive and specific detection of an infectious agent or a transgene or allele or non-encoding sequence of particular interest, in any type of biological sample, including especially in cases of newborns or immunocompromised subjects, the kit being of simple and feasible use in any infrastructure or equipment condition, as well as ideal to be used under field conditions.

The present kit uses a method that is based on the isothermal amplification of a nucleotide template under analysis by means of the "Loop Mediated Isothermal Amplification" (LAMP) technique, developed by Notomi et al. (2000), See patent US 6410278, Notomi T. and Hase T., "Process for synthesizing nucleic acid", published on June 25, 2002. This technique has already been successfully applied in different countries for the diagnosis of different pathologies such as AIDS and malaria, in most cases, by means of 'in house' reagents. It involves an isothermal reaction, for which reason it does not require any complex equipment, but only some thermal device of any kind.

The detection method of the present invention is extremely specific and sensitive but also robust and extremely easy to use, hence it does not require either any complex equipment or infrastructure or highly qualified human resources, for which reason it falls under the POC (Point of Care) diagnosis.

Furthermore, the kit of this invention is specially directed to solve problems in the detection of nucleic acid sequences in samples where serologic methods cannot be applied or are not sufficiently sensitive, such as those obtained from immunocompromised patients and newborns.

The provision of the sample and its adaptability to be used as a molecular template is extremely simple. A great innovation of this kit is that a biological sample of blood, tear, urine, etc. is directly placed in the reaction tube, with or without additional dilution, or is previously placed on a carrier, for example filter paper, and directly introduced in the amplification reaction without any prior purification steps, thereby avoiding complex steps that imply a longer period of time to get the result, the risk of losing or contaminating the biological sample in the process, the need to rely on a laboratory with certain degree of complexity and qualified human resources as well as higher costs.

### OBJECTS OF THE PRESENT INVENTION

It is an object of the present invention to provide an extremely easy-to-use sensitive and specific kit for detection of DNA or DNA copy or RNA fragments from pathogenic agents, transgenes, alleles or non-encoding sequences of interest, without the need of prior preparation or isolation of the nucleotide material.

Another object of the present invention is to provide a kit that does not require the use of automatic pipettes, tips or thermal cyclers, nor any other sophisticated laboratory equipment.

Another object of the present invention is to provide an easy-to-use kit for detection of DNA, DNA copy or RNA fragments suitable for use in "POC" ("Points of Care"), inside and outside laboratories with a great number of instruments.

Another object of the present invention is to provide an easy-to-use kit for detection of DNA, DNA copy or RNA fragments, wherein the reagent mixture provides sensitivity levels equal to or higher than the sample extraction and amplification systems available in the art.

Another object of the present invention is to provide an easy-to-use kit for detection of DNA, DNA copy or RNA fragments, wherein the reagent mixtures provide formulations that allow for keeping them stable under cooled conditions for periods of time of at least 18 months.

### BRIEF DESCRIPTION OF THE DRAWINGS

Figure 1 shows analytic sensitivity of the kit intended to detect Trypanosoma cruzi, the agent causing Chagas disease with purified genomic DNA.
Figure 2 shows the limit of the Chagas kit in blood samples without purification.
Figure 3 shows analytic specificity of the kit with DNA from the six DTUs of T. cruzi.
Figure 4 shows analytic sensitivity of the kit for syphilis.
Figure 5 shows a display of the result with visible light and UV of the detection of treponema with syphilis confirmed diagnosis.
Figure 6 shows analytic sensitivity of the kit for detection of SARSCoV-2, with reading from direct display with the naked eye from genomic DNA (reference sample) isolated from the nasopharyngeal swab of a confirmed patient by the reference method (rt-PCR and clinical condition).
Figure 7 shows analytic sensitivity of the kit for detection of SARS CoV-2 with a reference sample and phylogenetically-related virus genomic template samples, spatial concomitance virus and potential contaminating genomes of the sample.
Figure 8 shows capability to detect SARS-CoV-2 using the kit on direct reference samples, of bucco-/naso-pharyngeal or saliva swab, inactivated by heat, and without carrying out any purification or additional extraction steps.

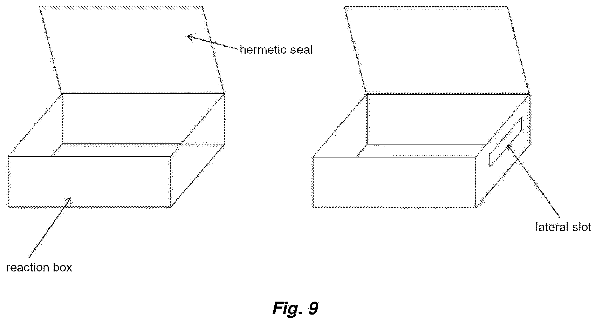

Figure 9 shows a perspective view of hermetically sealed sample boxes.

### DETAILED DESCRIPTION OF THE INVENTION

The present invention consists of an extremely easy-to-use molecular-based diagnostic kit for detection of specific nucleotide sequences such as DNA, DNA copy and RNA, useful for detecting infectious agents, transgenes, specific alleles and non-encoding sequences, the kit comprising the following components:
- a first calibrated dropper bottle with an anti-contamination system containing a solution of the isothermally-functioning polymerase enzyme, the 4 types of deoxynucleotides, i.e. dATP, dCTP, dGTP and dTTP, saline and buffer solutions, chaotropic agent betaine, and a non-ionic detergent.
- a second calibrated dropper bottle with an anti-contamination system containing at least a set of 4 or 6 necessary primers to multiply DNA, DNA copy or RNA fragments, the latter being, in a first step, undistinguishable within the reaction passed to DNA copy, the specific sequence to be detected, i.e. pathogenic agent, transgene, allele, etc.;
- as an alternative to the two dropper bottles mentioned above, a single calibrated dropper bottle may be provided, said bottle with an anti-contamination system, containing a solution with the 4 types of deoxynucleotides, i.e. dATP, dCTP, dGTP y dTTP, saline and buffer solutions, chaotropic agent betaine, a detergent, and the set corresponding to 4 or 6 primers, wherein the polymerase enzyme is lyophilized in the interior of the hermetically-sealed carrier where the amplification reaction is to be carried out;
- an alternative embodiment with a single calibrated dropper bottle containing a solution of the 4 types of deoxynucleotides, i.e. dATP, dCTP, dGTP and dTTP, saline and buffer solutions, chaotropic agent betaine, a detergent, the Bst enzyme and, in case of detection of RNA virus, the reverse transcriptase; wherein the primers and the indicating dye are dehydrated in the interior of the hermetically-sealed carrier where the amplification reaction is to be carried out;
- optional embodiment: in an optional embodiment of the kit, hermetically-sealed tubes or carriers may be included, in which the reagents with the sample are placed and the reaction is carried out;
- a third dropper bottle with developing solution;
- thin layer chromatography strips by lateral flow or simply reactive lateral flow dipsticks (LFD);
- for embodiments with color or fluorescence reading, LFDs are omitted; additionally, when the dying and/fluorescent compound is found in one of the reaction mixtures of the calibrated dropper bottles or else dehydrated in the reaction tube, the developing dropper bottle is also omitted;
- to verify correct kit performance, a set of positive and negative controls is provided, namely:
- positive control: consisting of a pattern sequence, that is an amplification template consisting of a nucleotide sequence equal to that intended to be detected, and
- negative control: consisting of the empty reaction carrier, such as a tube or filter paper in the tube, with no specific template; alternatively, the carrier is inoculated with a control genome not homologous to the sequence to be detected.

The calibrated dropper bottles that are used for the reagent mixtures must comply with the design standards such that there is no sterility loss during their use.

Eventually, they may be replaced by common dropper bottles with calibrated drop provided a preservative is added to the reactive. Preservatives may be selected from the group consisting of commercially-available preservatives or others known in the prior art: benzyl alcohol, cetrimide, benzalkonium chloride, cetylpyridinium chloride, benzethonium chloride, benzoic acid and salts thereof, sorbic acid and salts thereof, parabens, phenoxyethanol, phenylethanol, disodium EDTA and others known in the prior art and mixtures thereof, the selection of which is made upon testing their performance in each particular case.

In this kit, one or two reagent mixtures are present, which are directly administered with a dropper bottle of sterile calibrated drops. This technology, being novel in kits of molecular biology or molecular diagnosis, allows for the addition of reagents in a simplified and effective manner.

In the present invention, particularly appropriate to detect pathogenic agents or else transgenic, allelic, non-encoding or own sequences in a sensitive, specific and simple manner, the biological sample is placed on a carrier, incubation takes place during 30 to 60 minutes at 62 to 64 ºC, and the result is revealed, by alternative means, depending on the sample type and case convenience, the entire procedure taking a time slightly longer than one hour. Among the reading options, those of color change and/or fluorescence, or else of the addition of a lateral flow dipstick (LFD) are found.

The dipstick is a developing alternative when the presence of color of the unprocessed sample, or the carrier, could interfere with the color or fluorescence signal of the reaction.

The first use steps of the diagnostic kit reveal small variations depending on the selected carrier; "carriers" being those elements where the sample to be analyzed is placed.

In one embodiment of the invention, the carrier is a reaction tube made of classic plastic material, for example, polypropylene or the like, that is hermetically sealed.

A second carrier is a hermetically sealed reaction tray where the sample is placed, optionally with a saline solution, and the reagents; it comprises a lateral slot through which the developing system is introduced by the dipstick.

It is made of a material that allows for heating thereof for reaction incubation.

A third carrier is a filter paper (Guthrie type or the like). A portion of the paper with the sample is embedded and is introduced into the reaction tube or tray to continue with the diagnostic process.

In those cases in which the sample carrier is a hermetically-sealed reaction tube or tray, the sample, i.e. blood, saliva, etc. must be placed into said carrier, whether tube or tray. Optionally, depending on the nature and condition of the sample to be tested, for example, whole liquid blood, an amount of saline solution may be placed to dilute the sample, when said action could enhance reaction sensitivity.

In case the sample carrier is a filter paper (Guthrie type or the like), the filter paper or the like must become impregnated with the sample, i.e. blood, tear, saliva, etc. The impregnated portion of the filter paper is placed into the reaction tube or tray, optionally an amount of saline solution may be added to dilute the sample, when said action could enhance reaction sensitivity.

Whatever the selected carrier may be, to proceed with the amplification reaction of the present invention, at least one drop from the first dropper bottle is placed into the reaction tube or tray and, if appropriate, at least one drop from the second dropper bottle is introduced into the reaction tube or tray. It is hermetically sealed and mixed until homogenization of the solution ingredients. It is incubated with a heat source at 63 - 65 ºC for 30 - 60 minutes; thereafter the heat source is removed.

In the embodiments of the invention including development by dipstick (LFD), once incubation is completed, a drop from the developing dropper bottle is added into the reaction tube or tray, and a dipstick (LFD) is introduced. In the case of a reaction tube, the dipstick is introduced through the tube mouth; in the case of a reaction tray, the dipstick is introduced through the lateral slot. The reaction mixture is allowed to progress by capillarity along the LFD for 2 - 5 minutes, the LFD is removed and the result is read, where 2 precipitation lines or bands is a reagent positive result; 1 precipitation line or band is a reagent negative result; no precipitation line or band is an invalid result.

In the case of a colorless sample, such as exudate, mucus, serum, etc., the developing system by dipsticks may be replaced by chromophore and/or fluorophore compound changing. To this end, the LFD system and its dropper bottle is substituted by the color reading system, which result may be detected with the naked eye and/or by UV irradiation fluorescence using a chromophore and/or fluorophore compound. Said compound may be found: in one of the calibrated dropper bottles, present during the amplification reaction; in an 'ad hoc' developing dropper bottle, where the developing solution is added at the end of the incubation; dispensed in dehydrated manner in the reaction carrier provided by the kit, on the base thereof or else on the lid, in which case it shall be mixed with the reaction product, by reversion, at the end of the incubation.

In cases where the material to be detected is of DNA or ribonucleic nature, a prior reverse transcription reaction independently of the kit may be carried out, or else the presentation of the kit that is adapted to said biological material may be used, because the reverse transcription followed by the amplification in a single operative step occurs.

### EXAMPLE 1

### Diagnostic kit for detecting the presence of Trypanosoma cruzi, Chagas disease etiological agent, in biological samples.

Chagas disease was originally limited to certain rural areas of Latin America. With climate change and present migration currents, the infectious agent is presently found in traditionally non-endemic, rural and urban areas, throughout the world.

Chagas detection in newborns is usually made by techniques of display of parasite Trypanosoma cruzi in the blood, such as microhematocrit, microStrout, etc. Only in certain premises with adequate infrastructure, human resources and economic conditions, eventually, PCR or Real time PCR techniques are also carried out, as from pure DNA. Similar procedures are applied to detection in immunocompromised subjects and in suspected acute cases.

The present kit for detecting Chagas disease has been conformed to "Point of Care" conditions, i.e. with scarce laboratory resources, and under field conditions. The kit has been adapted to be applied using a blood sample obtained from the newborn heel and dispensed on a filter paper, or else from a patient's anticoagulated blood with guanidium chloride - EDTA or with EDTA only. The kit can also be used with pure DNA or from other biological samples, for example, patient's cerebrospinal fluid with reactivated Chagas disease. In this kit, the polymerase enzyme is Bst 2.0.

The Bst enzyme family is formed by DNA polymerase/helicase, using in this invention those without exonuclease activity such as Bst large fragment, Bst 2.0 and Bst 3.0, which features can be found in: FAQ: When should Bst DNA Polymerase be the enzyme of choice?, New England BioLabs Inc., 240 County Road, Ipswich, MA 01938-2732, USA

(https://www.neb.com/faqs/0001/01/01/when-should-bst-dna-polymerase-be-the-enzvm e-of-choice), and Bst DNA Polymerase - Heat-resistant strand displacement DNA polymerase, Nippon Gene CO., Ltd., 2-7-18, Toiya-machi, Toyama 930-0834, Japan (http://nippongene.com/english/product/modifying-enzvmes/bst-dna20

### polymerase.html).

In this kit for detecting Trypanosoma cruzi, the set of primers consists of 6 oligonucleotide sequences:
1- Forward Inner Primer (FIP): genetically-engineered sequence not found in nature.
2- Forward Outer Primer (F3): genetically-engineered sequence coupled with an immunoreactive protein, for example, biotin or fluorescein, that it is not the protein coupled in the B3.
3- Forward Loop Primer (FLP): sequence present in the loop formed in the 5' region of the amplicon. Under unfavorable reaction conditions - for example, due to excess of certain serum proteins in the sample - the presence of this primer enhances sensitivity, its presence being indistinct under other conditions.
4- Backward Inner Primer (BIP): genetically-engineered sequence not found in nature.
5- Backward Outer Primer (B3): genetically-engineered sequence coupled with an immunoreactive protein, for example, biotin or fluorescein, that it is not the protein coupled in the F3.
15- Backward Loop Primer (BLP): sequence present in the loop formed in the 3' region of the amplicon. Under unfavorable reaction conditions - for example, due to excess of certain serum proteins in the sample - the presence of this primer enhances sensitivity, its presence being indistinct under other conditions.

In this kit, the developing system is that of LFD type dipsticks, of the type of those described in patent US 6656744, with 2 areas of molecular interaction, each of which being capable of exclusively interacting with one or other immunoreactive protein coupled to primers F3 and B3.

In this kit, the developing solution consists of Tris buffered saline, which provides molecules coupled to colloidal gold solution, supplementary to the immunoreactive proteins coupled to primers, and facilitates the reaction progress over the developing stick by capillarity and which ionic strength and pH facilitate the interaction of immunoreactive proteins coupled to both primers with the corresponding molecules supplementary to the stick and the solution.

Analytical tests have shown sensitivity of 0.1 femtograms of DNA, equivalent to 0.001 parasites, either in solution or dispensed on filter paper (Guthrie type or the like), as well as the detection of one (1) parasite in a blood sample artificially inoculated and dispensed on filter paper and directly used in the reaction. The kit showed excellent performance in specificity, without evidencing crossed reaction with genetic material of other phylogenetically related species, such as Leishmania spp., Trypanosoma brucei, Trypanosoma Rangeli, or the like, that are contaminants to the sample, such as human DNA, yeast DNA.

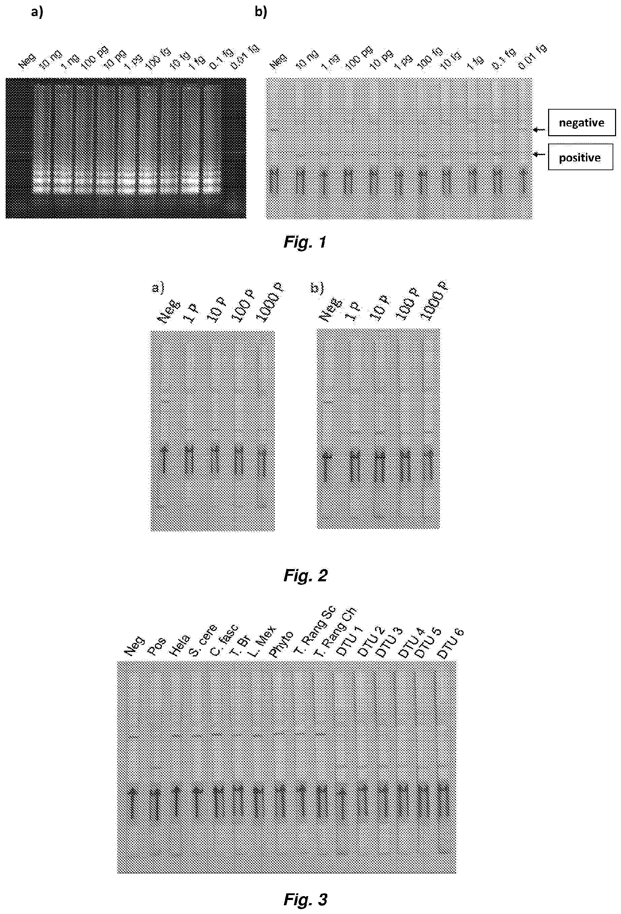

According to Figure 1, analytical sensitivity of this kit with purified genomic DNA may be observed.

The detection limit of the reaction with the kit was observed using serial 10-fold dilutions from 10 nanograms to 0.01 femtograms of purified genomic DNA as of T. cruzi, CL Brener strain, DTUVI (DTU "Discrete Typing Unit") VI. Similar results are obtained with strains of all DTUs:
a) Development through agarose gel electrophoresis 1.5% w/v, TAE 1X, Sybr Safe 1X (analytical method).
b) Development with the POC method using the LFD sticks of the kit.

The upper arrow defines the control line or band, present in all reactions, whether positive or negative, and the lower arrow corresponds to positive amplification band, where, Neg: control of reagents without genomic DNA, which must always be negative.

In Figure 2, the detection limit of Trypanosoma cruzi, the agent causing Chagas disease tested in blood samples without purification, dispensed on paper (Guthrie type or the like), inoculated with a controlled number of parasites, is shown.

Upon applying the kit directly in non-purified human blood samples artificially inoculated with a controlled number of whole parasites, among 1 and 1,000 parasites, dispensed on paper (Guthrie type or similar), optimal analytical sensitivity is observed, that is the detection of only one parasite per reaction.
a) samples inoculated with T. cruzi epimastigotes, CL Brener strain.
b) samples inoculated with CL Brener tripomastigotes.

Similar results are obtained with all DTUs: where, P: number of inoculated parasites; Neg: control of reagents without template, which must always be negative.

In Figure 3, tested analytical specificity of the kit with DNA of the six DTUs of T. cruzi, related species and contaminating species, may be observed.

Tests of the kit with DNA of the six DTU of T. cruzi; human cells (HELA), since it is a material present in the samples; yeast Sacharomicies cereviciae (S. cere), a frequent contaminant; and of species related to the parasite: Crithidia fasciculata (C.fasc), T. brucei (T. Br); Leishmania mexicana (L. Mex), Phytomonas spp (Phyto), T. rangeli SC-58 strain (T. Rang Sc) and T. rangeli Choachi strain (T. Rang Ch).i, were informed; where Neg: control of reagents without genomic DNA, which must always be negative; Pos: control of amplification using 1 pg of genomic DNA, which must always be positive.

These results confirm specificity of the kit to recognize all Trypanosoma cruzi genetic variants and that no crossed reaction occurs with other species, including those very similar due to their phylogenetic proximity.

### EXAMPLE 2

### Diagnostic kit for detecting the presence of Trypanosoma pallidum pallidum, etiological agent of Syphilis, in biological samples.

Syphilis is a sexually transmitted bacterial infection (STI) caused by Treponema pallidum pallidum. Despite having safe prophylactic measures, such as, for example, the use of condoms, and efficient and economic therapies, for example, using penicillin, this disease constitutes a global problem, with at least 12 million new cases of infection each year. Either in cases of syphilis in adults as in congenital syphilis, where late diagnosis and treatment occur, bacterial dissemination in the whole body is seen, which may result in neurologic and cardiovascular disorders.

Syphilis diagnosis is currently made by:
i- Microscope observation of T. pallidum;
ii- "Treponomic"-type serological methods, with which antibodies against specific antigens of T. pallidum are detected; and
iii- Non-treponomic type serological methods, with which antibodies against reaginic antigens, existing on damaged tissue, either by T. pallidum or for other reasons are detected, the positivity of which is not confirmed.

These methods for detecting syphilis, in general terms, are not effective in newborns, for which reason most alive, infected newborns (-80%) -that are asymptomatic at the time of birth- will not be detected early.

The methods of molecular detection of syphilis as well as Chagas disease developed up to the present time do not adapt to the general conditions found in the Public Health institutions of Argentina, for which reason they remain restricted only to certain medical care centers, and leave a vacancy for new specific, sensitive methods applicable to all sanitary conditions.

The kit of the present invention for detecting syphilis is adapted to Point of Care conditions. In this kit, as described in Example 1, polymerase enzyme is also Bst 2.0, and the set of primers is formed by 6 specific oligonucleotides of the Treponema pallidum pallidum sequence to be detected (FIP; F3; FL; BIP; B3 and BL).

In this kit for detecting treponoma in a colorless sample of, for example, exudate, mucus, serum, etc., the result reading system is made by color change which may be detected with the naked eye and/or UV irradiation fluorescence, using the intercalating compound "Sybr Green". To ensure stability of the reading system in this disclosure, said compound has been dispensed on the lid of the reaction tube provided in the kit, under dehydrated condition, and it is mixed with the reaction product at the end of incubation at 63 - 65 ºC.

Alternatively, for detection of the treponema in samples with opacity or their own color, or else dispensed on a filter paper, the developing system is that of LFD dipsticks as the kit of the Example 1.

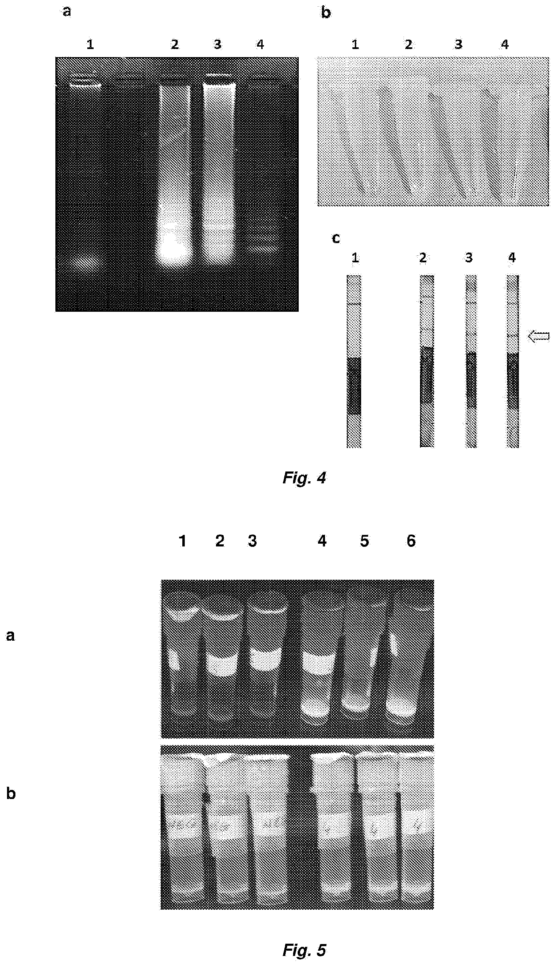

In Figure 4, analytical sensitivity of the kit for syphilis in samples of serum artificially inoculated can be observed, using different methods displaying the result.

The reaction with the kit was made to detect treponema in human serum samples 1/100 diluted, artificially inoculated with a controlled number of copies of treponema template of interest (among 1 and 100 copies) dispensed in reaction tubes. Optimal analytical sensitivity is seen, that is the detection of only one bacterium, in the different reading methods:
a) analytical method, through agarose gel electrophoresis;
b) color change; and
c) reading method through LFD.

In this way, lane 1: negative control corresponding to 1 picogram of E. coli DNA, which represents approximately 200 genome copies; lane 2: serum dilution with 100 copies of T. palludum nucleotide sequence used as template; lane 3: serum dilution with 10 template copies; and lane 4: serum dilution with 1 template copy in the reaction.

In Figure 5, the results obtained through the kit for detecting treponema in control serum samples and in a patient with confirmed diagnosis of syphilis by means of visible light and UV display, are seen.

In this Figure 5, 3 negative reactions are observed, as follows: 1, 2 and 3: carried out with non-infected serum, artificially inoculated with 1 picogram of E. coli DNA, approximately 200 genome copies, and 3 positive reactions, namely: 4, 5 and 6: triplicates of a reaction made with a 1/1000 dilution of a serum sample from a patient with confirmed infection, seen with the naked eye or else by UV light irradiation. Under controlled conditions, the kit provides, with both readings, an analytical detection limit of a copy per reaction.

To assess the number of copies present in the serum sample from the patient, complementary molecular assays by means of Real Time PCR with serial dilutions of the sample and the cloned sequence, used as positive control, were made.

These results show that the kit provides specific amplification for T. palludim pallidum, since no false positives are obtained in negative or DNA inoculated controls of other bacteria, of human or yeast origin. The detection limit obtained, of only one copy of DNA template per reaction, is similar to the different reading methods, the method of color change being very appropriate for POC conditions. In the literature, the reading system with UV is found to be more sensitive than that with visible light; however, when applying this kit under controlled conditions, the above-mentioned difference is not seen, probably given to the high efficiency of amplification for which, having only one initial copy, the amplification product is sufficiently high to be detected by both reading forms with substantially the same sensitivity.

### EXAMPLE 3

### Diagnostic kit for detecting the presence of SARS- CoV-2, COVID-19 agent, in biological samples.

This kit detects the infection caused by SARS-CoV-2, etiological agent of atypical pneumonia (COVID-2019, "Corona Virus Disease 2019"), by reverse transcription followed by molecular amplification of specific regions of the SARS-CoV-2 genome, in only one step, from isolated RNA or directly from a clinical sample, without any extraction/purification steps, from infected patients, and inactivated at 65 ºC - 30 minutes as suggested by the American Type Culture Collection (ATCC) 10801 University Boulevard, Manassas, VA 20110 USA (https://www.atcc.org/en/Global/Products/VR-1986HK.aspx#characteristics).

This diagnosis is presently made by:
i) Serological methods: these methods may be conventional, such as those conducted at a biochemical laboratory with commercial or homemade kits, or else kits of rapid configuration. These tests, apart from being rapid, are relatively inexpensive and do not need complexity in infrastructure or equipment. However, they may lead to false negative cases, because there is a period of time between the infection and the appearance of specific antibodies.
ii) Molecular methods, by end point PCR and Real Time PCR: These methods, as discussed above, require certain degree of infrastructure, equipment and skilled human resources. The process of taking the sample, isolating the viral RNA, carrying out the amplification and analyzing/informing the result takes, in the best optimized cases, some 4 - 5 hours. To this, in many cases, the time of sample transportation from primary medical centers, where the sample collection takes place, to sites qualified to carry out the diagnosis by Real Time PCR, must be added. In the case of Argentina, delays of 6 - 7 days have been reported, with the resulting negative impact on the patient's life and their family of staying preventively isolated until having the result.

Recently, numerous kits of serological and molecular basis have appeared all over the world; among the latter, those 3 LAMP-based kits appear, with different configuration.

Ben Assa et al. ("SARS-CoV-2 On-the-Spot Virus Detection Directly From Patients", Elect Jour Art, medRxiv, Cold Spring Harbor Laboratory Press,10.1101/2020.04.22.20072389 (2020)) discloses an isothermal molecular method similar to that of the present invention. Although it is estimated that it can be used directly with the RNA sample without purification, matching values obtained in comparison with the RT-PCR method (standard method for these determinations) have a very high percentage of false negatives of more than 15% in all cases, even with an improved protocol. This may be because this kit has a very high detection limit compared to RT-PCR. When comparing this method with the one provided with the kit described in the present invention, it may be stated that the unexpected result obtained as to the match obtained with an RT-PCR used as a reference method by 'ANLIS (National Bureau of Laboratories and Health Institutions) Malbrán' of the Argentine Republic, may be concluded.

A molecular-based diagnostic kit based on isothermal nucleic amplification for COVID-19 has been approved and is already being marketed by Abbott Laboratories. In this case, the virus gene is only amplified, which implies that the negative results may only be reported as 'presumptive' and another kit is required to confirm them. Moreover, the detection limit is of 312.5 copies, that is 125 copies/mL in 2.5 mL of elution buffer. This kit, contrary to the kit of the present invention, requires equipment provided by Abbott. The kit of the present invention does not require any special equipment or higher technology, but only a thermal block.

On the other hand, document US 2020/0048722 A by Nyan, D. discloses multiple diagnosis methodology by isothermal nucleic amplification that detects pathogen presence by fluorometry. The present invention does not require either a fluorometer or any other type of equipment since detection is visual based on the color change with the naked eye.

In summary, these commercially-available kits either require technology that is not easily available or is sophisticated such as cyclers, fluorometers, etc., or else have a very lower sensitivity compared to the standard molecular methods of the RT-PCR type. Thus, it is useful to have a rapid, sensitive and specific kit that does not require high technology equipment to perform disease diagnosis even in health centers with a reduced number of equipment or in the field. The present invention appears to overcome this particularly required need in the case of pandemics where instruments and supplies are scarce, and wherein a "Point of Care" diagnostic reagent, with an adequate sensitivity to allow for taking of decisions of immediate isolation or relocation of patients to avoid contagions and propagation of diseases, is required.

With the kit of the present invention, SARS-CoV-2 is detected by means of a reverse transcription reaction to obtain viral DNAc followed by molecular amplification of only one operative step, at constant temperature of 63 - 65 ºC. Detection is multiple because 4 sets of primers that recognize and amplify 4 specific regions of the viral genome are used, ensuring sensitivity even in the light of a mutation that may, potentially and unlikely, affect the hybridization of some primers. For simplified reading, hydroxynaphthol blue (HNB) is used, an indicating dye that changes from violet to blue when amplification reaction is positive.

Said change is due to the change of Mg2+ free in solution as dNTPs decrease and the pyrophosphate product of the amplification increases.

In this kit, the HNB dye together with primers are dehydrated on the bottom of the reaction tube provided by the kit. These components are suspended with reaction mixture and the template, and then incubation at 63 - 65 ºC takes place.

Analytical tests show sensitivity of 3.12 copies of SARS-CoV-2 viral genome from purified genomic RNA (reference sample).

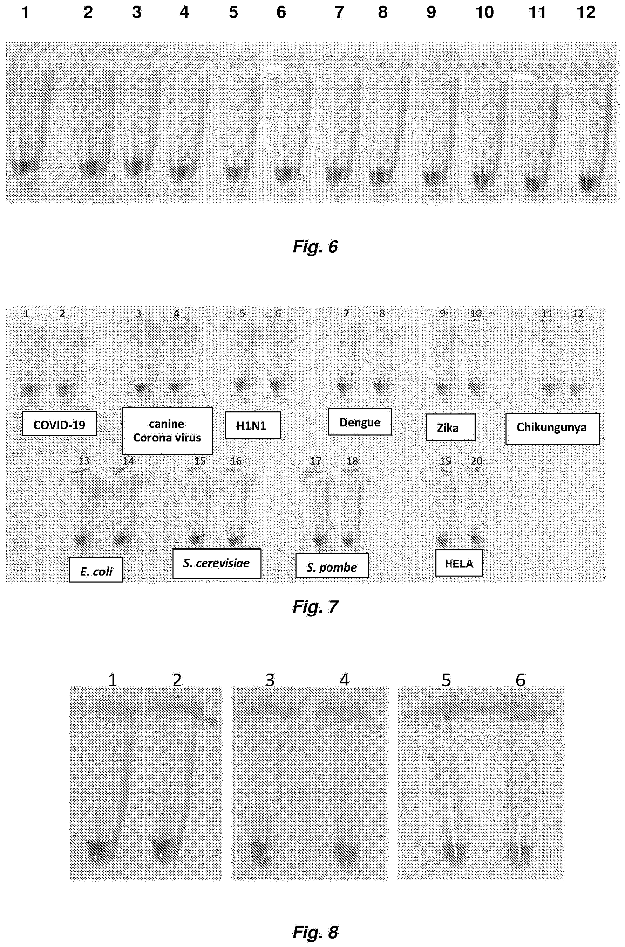

In Figure 6, analytical specificity of the kit for detection of SARS-CoV 2 according to the present invention can be observed by direct reading with the naked eye.

Successive dilutions were made from a reference sample of RNA isolated from naso-/bucco-pharyngeal swab. Tube 1: negative control, without template; tube 2: 40,000 copies; tube 3: 4,000 copies; tube 4: 400 copies; tube 5: 200 copies; tube 6: 100 copies; tube 7: 50 copies; tube 8: 25 copies; tube 9: 12.5 copies; tube 10: 6.25 copies; tube 11: 3.125 copies; tube 12: 1.56 copies of genome.

The kit discloses suitable specificity because no crossed reaction is seen using as a potential template the genetic material from other phylogenetically related species, such as, for example, canine coronavirus of spatial concomitance, such as H1N1, dengue, zika, chikungunya; neither with potentially contaminating genomes of the sample, such as bacteria, yeast and human's, using in all cases a mass 1,000 times greater than that used for SARS-CoV-2.

In Figure 7, analytical specificity of the kit for detection of SARS-CoV 2 may be observed, challenged with genome samples from related species and contaminating species.

In this Figure 7, duplicates of the reaction with different templates are shown: tubes 1-2: SARS-CoV-2; tubes 3-4: canine coronavirus; tubes 5-6: Influenza (H1N1); tubes 7-8: Dengue, mixture of the 4 serotypes; tubes 9-10: Zika; tubes 11-12: Chikungunya; tubes 13-14: bacterial template of E. coli; tubes 15-16 yeasts (Saccharomyces cerevisiae); tubes 17-18: yeasts (Saccharomyces pombe); and tubes 19-20: human cells (HELA).

Trials of the test were made using clinical bucco/naso-pharyngeal samples and saliva swab as template, inactivated at 65 ºC - 30 minutes as per indication of ATCC (American Type Culture Collection), with no extraction or purification steps. The kit shows amplification capability, either for swab sample as for saliva samples, for which reason it may be inferred that in sputum samples a positive result may likewise be obtained (assay not carried out). Inactivations were also conducted at 95 ºC - 5 minutes as indicated for Ebola in the General Procedures for the Inactivation of Potentially Infectious Samples with Ebola virus and other Highly Pathogenic Viral Agents, 2014-cha-procedimientos-inactivacion-ebola (https://www.paho.org/hq/dmdocuments/2014/2014- cha-procedimientos-inactivacion-ebola.pdf) attaining the same inactivation result.

In Figure 8, the reaction result with the kit for detection of SARS-CoV 2 may be observed, applying direct clinical samples, without purification inactivated by heat.

A group of swab samples and of saliva of patients which were confirmed positive and negative, wherein the samples of these latter were used as a negative control, were inactivated by heat (65 ºC - 30 minutes); and were used as template for direct reaction of the kit for COVID-19. Tube 1: swab sample from negative patient; tube 2: swab sample from positive patient; tube 3: saliva sample from negative patient, 1/10 dilution in saline solution; tube 4: saliva sample from positive patient, 1/10 dilution in saline solution; tube 5: negative control, without template; and tube 6: positive control based on RNA of reference SARS-CoV-2.

### EXAMPLE 4

### Determination of the detection limit of the kit for detecting SARS CoV 2.

To determine the detection limit, each operator (two in total) made repetitions of the reaction of the kit according to the present invention for COVID-19 with different amounts of purified RNA corresponding to the reference sample for SARS-CoV-2. In parallel, repetitions of a negative reference sample were made. The results summarized in the following table were obtained:

| Reactions | Positive reference sample (amount of copies of genome inoculated as template) | | | | | | Negative reference sample |
|---|---|---|---|---|---|---|---|
| | 100 Copies | 50 copies | 25 copies | 12.5 copies | 6.25 copies | 3.125 Copies | |
| Positive | 10 | 10 | 10 | 10 | 8 | 5 | 0 |
| Negative | 0 | 0 | 0 | 0 | 2 | 5 | 10 |
| % | 100 | 100 | 100 | 100 | 80 | 50 | 100 |

It is concluded that the detection limit of the kit is 12.5 copies of SARS-CoV-2 RNA.

### EXAMPLE 5

### Comparison of the kit for detecting SARS-CoV-2 of the present invention versus RT-PCR kits with samples coming from the national reference center of the Argentine Republic "ANLIS Malbrán".

### Parameter Definition

| | **Reference technique RT-PCR** | |
|---|---|---|
| | **Positive samples** | **Negative samples** |
| **Kit of the invention Positive** | VP | FP |
| **Kit of the invention Negative** | FN | VN |

| | | |
|---|---|---|
| VP: True Positive; VN: True Negative; FP: False Positive; FN; False Negative. | | |

Based on this picture, the following parameters of the kit are defined:
**Sensitivity** (%); VP / (VP+FN) x 100
**Specificity** (%): VN / (VN+FP) x 100
**Accuracy** (%): (VN+VP) / total x 100

### Study of sampling under clinical and trial conditions with the kit of the invention COVID-19 under controlled conditions.

150 clinical reference samples were received, consisting of purified RNA from patients diagnosed by the RT-PCR of the "gold standard" method of COVID-19 emergency according to the WHO, 100 coming from positive patients and 50 from negative patients. Likewise to the RT-PCR, 5 µl of purified RNA by a commercial kit for the extraction of nucleic acids by microcolumn (microcolumns identified as RNA spin Qiagen, or similar) as template for the test of the kit of the present invention COVID-19, were used.

Results obtained in both methods are compared on a contingency table of 2 x 2, defining the performance parameter, sensitivity and specificity of the kit of the present invention COVID-19.

### Results

Results obtained in the 150 clinical samples tested by the kit of the present invention for COVID-19, taking the RT-PCR as a Gold Standard method of COVID-19 emergency, show a 100% matching, for which they evidence 100% sensitivity and 100% specificity (Figures 5A and B), as shown in the following table:
Parameter Definition

| | **Reference technique RT-PCR** | | **total** |
|---|---|---|---|
| | **Positive samples** | **Negative samples** | |
| **Kit of the invention Positive** | VP | FP | |
| **Kit of the invention Negative** | FN | VN | |

| | | | |
|---|---|---|---|
| VP: True Positive; VN: True Negative; FP: False Positive; FN; False Negative. | | | |

Based on this picture, the following parameters of the kit are defined:
**Sensitivity** (%); VP / (VP+FN) x 100
**Specificity** (%): VN / (VN+FP) x 100
**Accuracy** (%): (VN+VP) / total x 100

100% matching as to negative samples was found. As to positive samples, there were 6 that showed non-matching, being positive for RT-PCR and negative for the kit of the present invention. These samples were repeated with the kit recommended by CDC 'Centers for Disease Control and Prevention', USA ("CDC 2019-Novel Coronavirus (2019-nCoV) Real-Time RT-PCR Diagnostic Panel", CDC-006-00019, Revision 04, CDC/DDID/NCIRD/ Division of Viral Diseases, 12/6/2020), the result matching with that of the present invention. Therefore, defective conservation of samples that caused degradation during their storage for more than 1 week, is evident. Taking this into account, the following table was made:

### Contingency table as per results forwarded bv the Referring 'ANLIS - Malbrán' and verified with CDC 2019-nCoV Real Time RT-PCR Diagnostic Panel

| | **Reference technique RT-PCR** | | **total** |
|---|---|---|---|
| | **Positive samples** | **Negative samples** | |
| **Kit of the invention Positive** | 94 | 0 | 94 |
| **Kit of the invention Negative** | 0 | 56 | 56 |
| **Totals** | 94 | 56 | 150 |

| | | | |
|---|---|---|---|
| Under these conditions, the following parameters are obtained: | | | |

**Sensitivity: 100 %**
**Specificity: 100 %**
**Accuracy: 100 %**

### EXAMPLE 6

### Inter-laboratory trial with the kit for detecting SARS CoV-2 of the invention with those obtained with RT PCR kits of different COVID-19 diagnostic centers of the Argentine Republic.

The following COVID-19 diagnostic centers have taken part in this inter-laboratory trial, which used RT PCR kits:

| **Diagnostic center** | **Location** |
|---|---|
| Rossi Hospital | La Plata, Province of Buenos Aires, AR |
| San Juan de Dios Hospital | La Plata, Province of Buenos Aires, AR |
| ANLIS Malbrán | Autonomous city of Buenos Aires, AR |
| San Fernando Hospital | San Fernando, Province of Buenos Aires, AR |
| Muñiz Hospital | Autonomous City of Buenos Aires, AR |
| Abete Hospital | Malvinas Argentinas, Province of Buenos Aires, AR |
| Public Health Laboratory, School of Exact Sciences, National University of La Plata | La Plata, Province of Buenos Aires, AR |

The total results obtained in the inter-laboratory trial are stated in the following contingency table:

### Contingency table

| | **Reference technique RT-PCR** | | **total** |
|---|---|---|---|
| | **Positive samples** | **Negative samples** | |
| **Kit of the invention Positive** | 201 | 6 | 207 |
| **Kit of the invention Negative** | 7 | 227 | 234 |
| **Total** | 208 | 233 | 441 |

**Sensitivity: 96.6 %**
**Specificity: 97.4 %**
**Accuracy: 97.0 %**

This inter-laboratory trial shows an extremely favorable matching and over 95% based on the three more relevant parameters of diagnostic kits. This is particularly remarkable if we take into account that in this trial:
- Several Argentine diagnostic centers took part.
- RT-PCR methods of different commercial trademarks, approved by the competent sanitary authority, i.e. National Administration of Drugs, Food and Medical Technology (ANMAT) of Argentina, such as: GeneFinder RealAmp SARS-CoV-2; RT-PCR Light Mix^{®} Modular SARS-CoV-2; or the like, were used.
- Samples, in general, could not be analyzed by the kit of the invention simultaneously with RT-PCR, for which reason some or all of the mismatches could be explained by some alteration of the integrity of the sample during storage. Besides, there exist other random variables, such as: inhomogeneity of the sample as to viral load, human mistake when making determination, etc.

### ORIGINAL PRIMER SEQUENCES https://www.epo.org/applying/online-services/online-filing/auxiliary/bissap.html

| **KIT** | | **SEQUENCES** |
|---|---|---|
| **1- kit for Chagas** | **SAT gene sequences** | F3- ACAGAGAGTGCCTCTGA |
| | | B3-GTCAATATCTGTTTGCG |
| | | FIP- CACTCGGCTGATCGTTTTCGACCCCCATCATTCATAATTGGA |
| | | BIP- GGCAAGAGCTCGCGAAATTCCACACACTGGACACCAAA |
| | | Lb- TCCAAGCAGCGGAT AATT C A |
| | | Lf- TGCATCACATGTTGTGGTCTA |
| | | |
| **2** - **kit for Syphilis** | **PolA gene sequence** | F3- ATTGGTCCTAAGACGGCT |
| | | B3- GCGGAATACAACAGGAATC |
| | | FIP- CAGCGCTTCTTTTAAGGAATAGGTAGCACATCTTCTCCACTGT |
| | | BIP- CGCACGAAGATAGTGTGTGGACATGGTACATCGTCACG |
| | | Lb- GAAGAAAGATGCATTTTTTTCTCGTTC |
| | | Lf- CGATAAATACCATCAAGTGTGCCAAA |
| | | |
| **3** - **kit for COVID-19** | **R1Aa2 sequences** | F3- TGCTTGTGAAATTGTCGGT |
| | | B3- GCCAGTTTCTTCTCTGGAT |
| | | FIP- TCAGCACACAAAGCCAAAAATTTATAAATTGTCACCTGTGCAAAG |
| | | BIP- TATTGGTGGAGCTAAACTTAAAGCCACACTTTCTGTACAATCCCTT |
| | | Lb- GAATTTAGGTGAAACATTTGTCACG |
| | | Lf- CTTAAAGAATGTCTGAACACTCTCC |
| | | F3-CCGACGACGACTACTAGC |
| | **pE-2 sequence** | B3- GACTCACGTTAACAATATTGCA |
| | | FIP- ACTATTAACGTACCTGTCTCTTCCGGTGCCTTTGTAAGCACAAG |
| | | BIP- AGCGTACTTCTTTTTCTTGCTTTCGCAGTACGCACACAATCGA |
| | | Lb-CTTGCTAGTTACACTAGCCATCCT |
| | | Lf- CGAATGAGTACATAAGTTCGTACTC |

## Claims

**1.** A molecular-based diagnostic kit for detection of nucleotide sequences, such as infectious agents, transgenes, alleles or non-encoding sequences, said kit comprising:
at least one first calibrated dropper bottle with an anti-contamination system that contains a solution of polymerase enzyme, saline ingredients, chaotropic agents and deoxyribonucleotides;
a second calibrated dropper bottle with an anti-contamination system that contains a set of at least 4 primers, with at least part of its sequences being preserved in the nucleic acid fragment to be detected;
a carrier to hold the sample to be tested, that can consist of absorbent paper that can be placed into a reaction tube or a plastic or metallic carrier, that is folded up over itself and can hold a sample and reagents in its interior, be closed and heated to afford the reaction and that, after incubation time, a dipstick for reading thereof can be introduced through the lateral part thereof;
at least one developing system by LFD ("Lateral Flow Dipsticks") or by colorimetric reaction; and
positive and negative controls,
wherein the polymerase enzyme is selected from the Bst enzyme family, which consists of DNA polymerase/helicase, with no exonuclease activity; and
wherein the set of primers is selected from the genome sequences or the fragment to be detected, whether of deoxyribonucleic or ribonucleic nature.

**2.** The diagnostic kit of claim 1, wherein the kit comprises at least a single calibrated dropper bottle with an anti-contamination system that provides a complete mixture of reagents, excluding the enzyme that may be dispensed in dehydrated state in each reaction tube.

**3.** The diagnostic kit of claim 1, wherein the kit comprises at least a single calibrated dropper bottle with an anti-contamination system that provides a complete mixture of reagents, excluding the indicating dye of the reaction that may be dispensed in dehydrated state in each reaction tube.

**4.** The diagnostic kit of claim 1, wherein the kit comprises at least a single calibrated dropper bottle with an anti-contamination system that provides a complete mixture of reagents, excluding the dye and primers that may be dispensed in dehydrated state in each reaction tube.

**5.** The diagnostic kit of claim 1, wherein said carrier that holds the sample to be tested is a reaction tube, tray or a filter paper (Guthrie type or the like).

**6.** The diagnostic kit of claim 3, wherein the reaction tray is a container having a hermetic seal and a lateral slot for dipstick introduction.

**7.** The diagnostic kit of claim 1, wherein said chromatographic sticks used in the developing kits in this system are LFD dipsticks comprising two bands or lines, a top one and a bottom one.

**6.** A method for detecting infectious agents, transgenes, alleles or non-encoding sequences, using the diagnostic kit of claim 1, wherein the sample carrier is a reaction tube, said method comprising the steps of:
placing the sample into a reaction tube,
placing at least one drop from the first dropper bottle into the reaction tube,
placing at least one drop from the second dropper bottle into the reaction tube,
covering the reaction tube,
mixing until homogenization of solution ingredients,
incubating at 63 - 65 ºC for 30 - 60 minutes,
removing the heat source,
adding one drop from the developing dropper bottle into the reaction tube,
introducing a LFD dipstick into the reaction tube,
allowing the contents of the amplification tube to progress by capillarity along the LFD for 2 - 5 minutes,
removing the LFD, and
reading the result,
wherein 2 bands is a positive result to the reagent, 1 band is a negative result to the reagent and no band is an invalid result, over a total of two bands, a top one and a bottom one on the LFD dipstick.

**7.** A method for detecting infectious agents, transgenes, alleles or non-encoding sequences using the diagnostic kit of claim 1, wherein the sample does not interfere with fluorescence or color reaction, said method comprising the steps of:
placing the sample into a reaction tube,
placing at least one drop from the first dropper bottle on the sample,
placing at least one drop from the second dropper bottle on the sample,
closing the reaction tube,
mixing until homogenization of the solution ingredients,
incubating at 63 - 65 ºC for 30 - 60 minutes,
removing the heat source,
adding a drop from the developing dropper bottle to the sample whenever the chromogenic or fluorogenic material was not included in the reaction, and
reading the result,
wherein color change or appearance of light emission by UV irradiation is a positive result, and no color change or the absence fluorescence is a negative result to the reagent.

**8.** A method for detecting infectious agents, transgenes, alleles or non-encoding sequences using the diagnostic kit of claim 1, wherein the sample does not interfere with fluorescence or color reaction, said method comprising the steps of:
placing at least one drop from the first dropper bottle into the reaction tube that contains essential ingredients for amplification and/or reading,
placing the sample into the reaction tube,
closing the reaction tube,
mixing until homogenization of the solution ingredients,
incubating at 63 - 65 ºC for 30 - 60 minutes,
removing the heat source, and
reading the result,
wherein color change or appearance of light emission by UV irradiation is a positive result, and no color change or the absence of fluorescence is a negative result to the reagent.

**9.** A method for detecting infectious agents, transgenes, alleles or non-encoding sequences using the diagnostic kit of claim 1, wherein the sample carrier is a filter paper (Guthrie type or the like), said method comprising the steps of:
placing the sample on the filter paper,
placing the filter paper impregnated with the sample into the reaction tube or tray,
placing at least one drop of the first dropper bottle on the sample,
placing at least one drop from the second dropper bottle on the sample,
closing the reaction tube,
mixing until the homogenization of the solution ingredients,
incubating at 63-65 ºC for 30 - 60 minutes,
removing the heat source,
adding one drop from the developing dropper bottle on the sample,
introducing a LFD into the reaction tube,
allowing the contents of the amplification tube to progresses by capillarity along the LFD for 2 - 5 minutes,
removing the LFD, and
reading the result,
wherein 2 bands is a positive result to the reagent, 1 band is a negative result to the reagent and no band is an invalid result, over a total of two bands, a top one and a bottom one on the LFD.

**10.** A method for detecting infectious agents, transgenes, alleles or non-encoding sequences using the diagnostic kit of claim 1, wherein the sample carrier is a reaction tray, said method comprising the steps of:
placing the sample into the reaction tray,
placing at least one drop from the first dropper bottle on the sample,
placing at least one drop from the second dropper bottle on the sample,
closing the reaction tray,
mixing until homogenization of the solution ingredients,
incubating at 63 - 65 ºC for 30 - 60 minutes,
removing the heat source,
adding one drop from the developing dropper bottle into the reaction tray,
introducing a LFD by the lateral slot until it contacts the sample,
allowing the contents of the reaction tray to progress by capillarity along the LFD for 2 - 5 minutes,
removing the LFD, and
reading the result,
wherein 2 bands is a positive result to the reagent, 1 band is a negative result to the reagent and no band is an invalid result, over a total of two bands, a top one and a bottom one on the LFD.
